(19) Europäisches Patentamt / European Patent Office / Office européen des brevets

(11) **EP 1 928 294 B1**

(12) **FASCICULE DE BREVET EUROPEEN**

(45) Date de publication et mention de la délivrance du brevet:
**08.09.2010 Bulletin 2010/36**

(21) Numéro de dépôt: **05798468.4**

(22) Date de dépôt: **18.08.2005**

(51) Int Cl.:
*A61B 3/107* *(2006.01)*    *A61F 9/008* *(2006.01)*
*A61B 3/12* *(2006.01)*    *A61B 3/103* *(2006.01)*

(86) Numéro de dépôt international:
**PCT/FR2005/002098**

(87) Numéro de publication internationale:
**WO 2007/020334 (22.02.2007 Gazette 2007/08)**

(54) **METHODE ET SYSTEME DE CORRECTION DES ABERRATIONS DE L' IL POUR INSTRUMENT OPHTALMIQUE**

VERFAHREN UND SYSTEM ZUR KORREKTUR VON ABWEICHUNGEN DES AUGES FÜR EIN OPHTHALMISCHES INSTRUMENT

METHOD AND SYSTEM FOR CORRECTING ABERRATIONS OF THE EYE FOR AN OPHTHALMIC INSTRUMENT

(84) Etats contractants désignés:
**AT BE BG CH CY CZ DE DK EE ES FI FR GB GR HU IE IS IT LI LT LU LV MC NL PL PT RO SE SI SK TR**

(43) Date de publication de la demande:
**11.06.2008 Bulletin 2008/24**

(73) Titulaire: **Imagine Eyes**
**91400 Orsay (FR)**

(72) Inventeurs:
• **LEVECQ, Xavier Jean-François**
**F-91190 GIF SUR YVETTE (FR)**

• **CHATEAU, Nicolas**
**F-75013 Paris (FR)**

(74) Mandataire: **Pontet, Bernard et al**
**Pontet Allano & Associés s.e.l.a.r.l.**
**25, Rue Jean Rostand**
**Parc Club Orsay Université**
**91893 Orsay Cedex (FR)**

(56) Documents cités:
**EP-A- 0 279 589        FR-A- 2 828 396**
**US-A1- 2004 160 576    US-B1- 6 271 915**
**US-B1- 6 601 956**

**Description**

L'invention concerne une méthode de correction des aberrations de l'oeil pour un instrument ophtalmique, un système de correction des aberrations de l'oeil pour la mise en oeuvre de ladite méthode ainsi que des instruments ophtalmiques équipés de tels systèmes de correction.

Les instruments ophtalmiques tels que par exemple les instruments d'imagerie rétinienne ou de traitement optique par laser sur la rétine fonctionnent avec un faisceau d'analyse destiné à traverser les divers éléments optiques (cornée, cristallin, ...) dont est formé l'oeil, soit en faisceau incident dans l'oeil (cas du traitement optique de la rétine), soit en faisceau émergent de l'oeil (cas de l'imagerie rétinienne). Dans tous les cas, les aberrations des différents éléments optiques de l'oeil entachent d'aberrations le front d'onde du faisceau d'analyse, ce qui dégrade la qualité de l'instrument optique. Ainsi dans le cas de l'imagerie rétinienne, l'image perd en résolution et, dans le cas du traitement optique de l'oeil, la qualité de focalisation du laser sur la rétine est dégradée. Il est connu d'associer à ces instruments ophtalmiques un système de correction des aberrations de l'oeil permettant de corriger le front d'onde du faisceau d'analyse, c'est-à-dire de donner à la phase du faisceau optique la forme la plus proche possible d'une forme prédéterminée permettant d'obtenir les performances optimales de l'instrument.

Un tel système comprend classiquement des moyens de mesure des aberrations de l'oeil, de type analyseur Shack Hartmann, et un dispositif optique de correction de la phase du front d'onde du faisceau d'analyse, de type miroir déformable, ou modulateur spatial de lumière, asservi au moyen d'une unité de commande pour corriger le front d'onde en fonction des aberrations de l'oeil mesurées. Un tel système peut aussi être utilisé dans un instrument de type instrument de simulation de vision, dont le but est de représenter à un patient les effets de différentes corrections (verres correcteurs, lentilles de contact, traitement optique de l'oeil) en lui faisant "voir" une image, le faisceau d'analyse incident dans l'oeil du patient étant alors corrigé des aberrations oculaires et/ou des effets induits par le phénomène que l'on souhaite simuler.

Cependant la correction des aberrations de l'oeil est limitée par les mouvements de l'oeil, mouvements latéraux, axiaux ou mouvements de rotation de l'axe oculaire, qui induisent des variations des aberrations de l'oeil à des fréquences d'évolution rapide (typiquement supérieures à 5 Hz). Ces variations d'aberrations liées aux mouvements oculaires peuvent être particulièrement gênantes dans certaines applications. Dans le cas de l'imagerie rétinienne par exemple, des images rétiniennes dites "super résolues" sont obtenues par combinaison d'images en coupe, de très petit champ, issues de la même zone de la rétine. Il est alors nécessaire d'avoir une très bonne stabilisation de l'image de la rétine sur le détecteur d'image pour effectuer la combinaison, malgré les mouvements de rotation de l'axe oculaire. De même, dans le cas du traitement optique de la rétine, les mouvements latéraux de la pupille ainsi que les mouvements de rotation de l'axe oculaire diminuent la précision de la focalisation du laser sur la rétine.

L'invention propose une méthode de correction des aberrations de l'oeil ainsi qu'un système pour la mise en oeuvre de la méthode, permettant d'améliorer la qualité d'un instrument ophtalmique, basée sur une mesure indépendante des mouvements de l'oeil et la prise en compte de cette mesure pour modifier la correction des aberrations.

Plus précisément, l'invention concerne une méthode de correction des aberrations de l'oeil appliquée à un instrument ophtalmique fonctionnant avec un faisceau lumineux d'analyse, comprenant:

- la mesure des aberrations de l'oeil susceptibles de perturber ledit faisceau d'analyse,
- la correction de la phase du front d'onde dudit faisceau d'analyse en fonction des valeurs mesurées desdites aberrations,
  **caractérisée en ce qu'**elle comprend en outre:
- la mesure des mouvements de l'oeil réalisée indépendamment de ladite mesure des aberrations,
- la modification de la correction de la phase du front d'onde du faisceau d'analyse en fonction de ladite mesure des mouvements de l'oeil.

L'invention concerne également un système de correction pour la mise en oeuvre de la méthode.

La méthode selon l'invention permet notamment, grâce à la mesure indépendante des mouvements de l'oeil, de prendre en compte en plus des aberrations intrinsèques de l'oeil (à basse fréquence d'évolution, typiquement inférieure à 1 Hz), les variations des aberrations liées aux mouvements de l'oeil, de fréquence d'évolution beaucoup plus rapide, pour obtenir une correction des aberrations à une fréquence au moins égale à cette fréquence d'évolution, sans pour autant travailler avec une fréquence élevée de l'analyseur de front d'onde, ce qui entraînerait une diminution inévitable de la résolution spatiale de l'analyseur.

D'autres avantages et caractéristiques de l'invention apparaîtront plus clairement à la lecture de la description, illustrée par les figures qui suivent:

- La figure 1, un schéma synoptique illustrant le système de correction selon l'invention.
- Les figure 2A à 2C, les schémas d'un instrument de type instrument d'imagerie rétinienne ou instrument de traitement oculaire équipé d'un système de correction, selon différents exemples de réalisation;

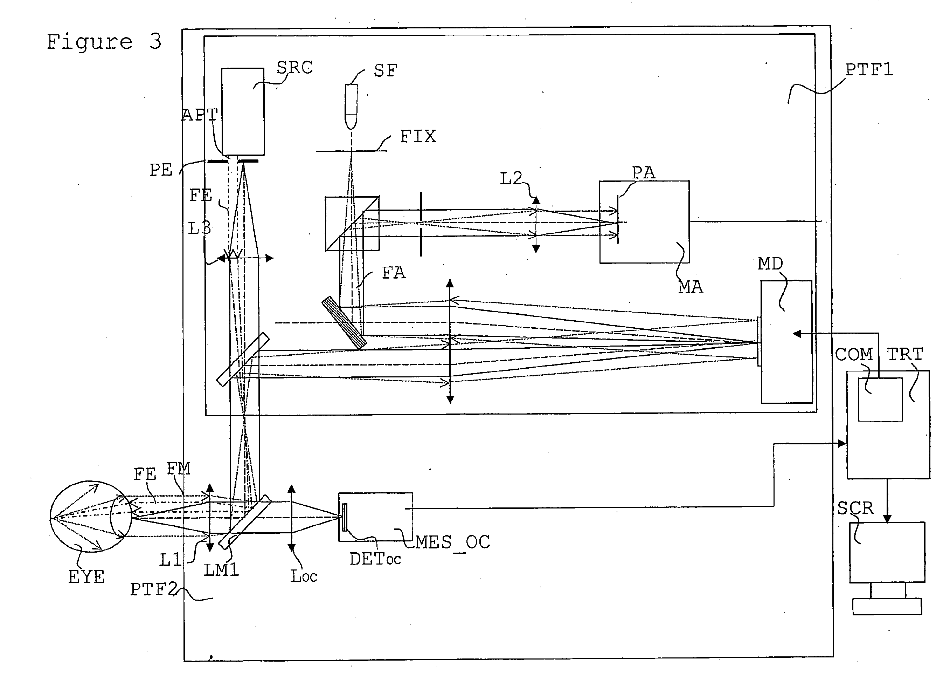

- La figure 3, le schéma d'un instrument de type instrument de simulation de la vision équipé d'un système de correction, selon un exemple de réalisation;
- La figure 4, un exemple de réalisation du dispositif de mesure des mouvements de l'oeil du système de correction selon l'invention;
- Les figures 5A et 5B, des images de la pupille formées par le dispositif de mesure de la figure 4, selon deux positions latérales de l'oeil ;
- La figure 6, un schéma illustrant la méthode de calcul de rotation de l'axe de l'oeil à l'aide du dispositif de mesure selon la figure 4;
- Les figures 7A à 7D, des schémas illustrant le calcul des déplacements axiaux de la pupille de l'oeil à l'aide du dispositif de mesure selon la figure 4.

Sur ces figures, les éléments identiques sont repérés par les mêmes références.

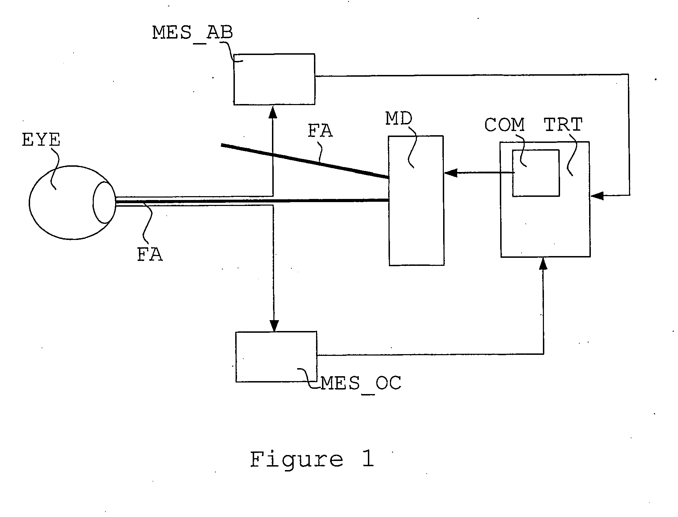

La figure 1 représente le schéma fonctionnel d'un système de correction selon l'invention, destiné à être intégré dans un instrument ophtalmique fonctionnant avec un faisceau lumineux d'analyse FA. Sur cet figure l'instrument lui-même n'est représenté que par le faisceau d'analyse FA, symbolisé sur la figure 1 par un trait fort, soit incident dans l'oeil EYE d'un patient (cas par exemple du traitement optique de la rétine par laser ou de la simulation de vision), soit émergent de l'oeil (cas de l'imagerie rétinienne). Le système de correction comprend d'une part des moyens de mesure MES_AB des aberrations de l'oeil susceptibles de perturber le faisceau d'analyse FA et un dispositif optique MD de correction de la phase du front d'onde dudit faisceau d'analyse, ainsi qu'une unité de commande COM du dispositif de correction avec des moyens de traitement TRT reliés aux moyens de mesure et calculant les valeurs de correction à appliquer au dispositif de correction en fonction des valeurs mesurées des aberrations. Sur la.figure 1, ces éléments ne sont pas représentés selon un schéma optique mais reliés par des flèches qui indiquent les liaisons fonctionnelles entre les éléments. Le système de correction selon l'invention comprend en outre un dispositif de mesure MES_OC des mouvements de l'oeil distinct des moyens de mesure des aberrations, les valeurs de mesures des mouvements de l'oeil étant envoyées à l'unité de commande afin de modifier la correction de la phase du front d'onde du faisceau d'analyse. Les performances de l'instrument ophtalmique se trouvent améliorées grâce à la mesure indépendante des mouvements de l'oeil et la prise en compte de cette mesure pour modifier la correction des aberrations.

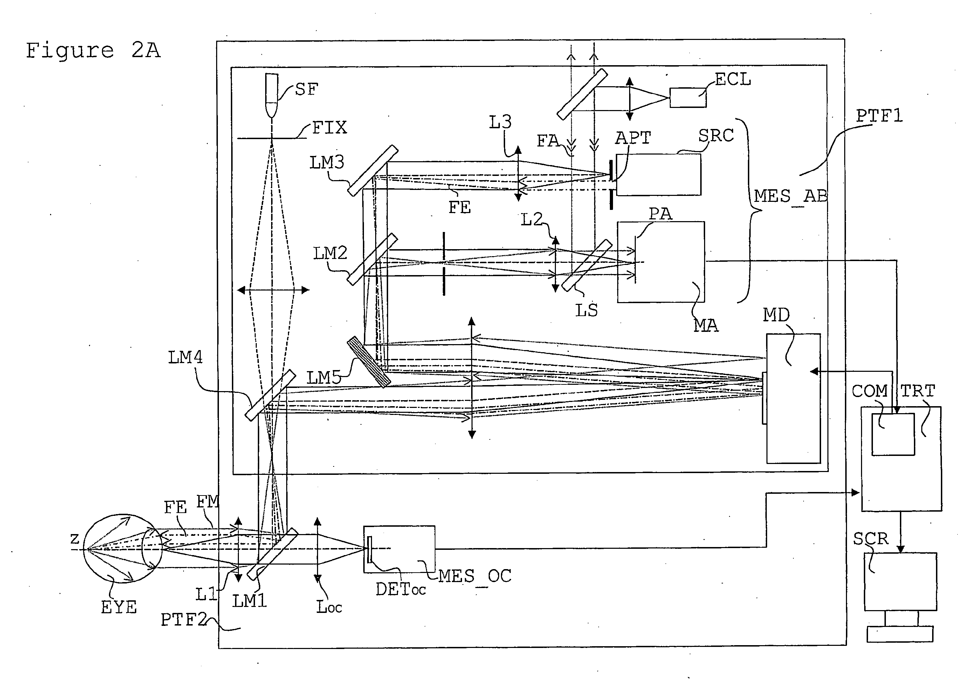

Les figures 2A à 2C représentent les schémas optiques d'un instrument de type instrument d'imagerie rétinienne ou instrument de traitement oculaire équipé d'un système de correction des aberrations de l'oeil selon l'invention, dans différents exemples de réalisation. Par soucis de clarté, sur ces schémas l'échelle n'est pas respectée. On entend par système d'imagerie rétinienne tout type d'instrument permettant la visualisation de la rétine quel que soit son mode de fonctionnement. Cela peut être par exemple un système de caméra fond d'oeil, un système d'angiographie, un instrument de type Scanning Laser Ophtalmoscope (SLO), ou de type Optical Cohérence Tomography (OCT).

Un instrument d'imagerie rétinienne comprend de façon classique un système d'éclairage ECL de la rétine permettant d'éclairer la rétine sur la zone que l'on veut imager, un système d'imagerie et de détection en sortie de l'instrument d'imagerie rétinienne recevant un faisceau d'analyse FA, rétro réfléchi par la rétine et émergent de l'oeil. Sur l'exemple de la figure 2A, l'objectif et le détecteur d'imagerie ne sont pas représentés. Les aberrations intrinsèques de l'oeil déforment le front d'onde du faisceau d'analyse et de ce fait diminuent la résolution de l'image. Classiquement, l'instrument d'imagerie rétinienne comprend également un système de correction des aberrations intrinsèques avec notamment des moyens de mesure MES_AB des aberrations de l'oeil, un dispositif optique MD de correction de la phase du front d'onde du faisceau d'analyse permettant de compenser les aberrations mesurées, et une unité de commande COM du dispositif de correction reliée aux moyens de mesure et comprenant des moyens de traitement TRT permettant de calculer les valeurs de correction à appliquer en fonction des valeurs mesurées des aberrations.

Un système de mesure des aberrations est décrit dans la demande de brevet français FR0111112. Il comprend notamment une voie d'éclairage avec notamment des moyens d'émission SRC d'un faisceau d'éclairage FE (en pointillé alterné sur les figures 2A à 2C) et un diaphragme d'éclairage APT pour former sur la rétine de l'oeil à analyser EYE un point source lumineux diffusant. Dans le cas de l'imagerie rétinienne, la zone éclairée par le système d'éclairage ECL est typiquement dix fois supérieure à la taille du point lumineux formé par le faisceau d'éclairage FE, et l'on fait l'approximation que les aberrations mesurées en ce point seront faiblement variables sur l'ensemble de la zone éclairée par le faisceau d'éclairage ECL. Avantageusement, les longueurs d'onde des faisceaux d'éclairage FE et du système d'éclairage ECL sont différentes de façon à simplifier la séparation optiques des faisceaux. Il comprend en outre une voie d'analyse avec notamment des moyens d'analyse optique MA dans un plan d'analyse PA donné de la phase du front de l'onde émise par ledit point source et émergente de l'oeil (faisceau FM représenté en pointillé sur les figures 2A à 2C). Des moyens d'imagerie L1 centrés sur un axe de mesure z correspondant à l'axe oculaire dans une position donnée de l'oeil, associé à des moyens d'imagerie L2 sur la voie d'analyse et L3 sur la voie d'éclairage permettent d'assurer la conjugaison optique entre la pupille de l'oeil, le plan du diaphragme d'éclairage APT et le plan d'analyse PA des moyens d'analyse MA. La lame séparatrice LM2 définit les voies d'analyse et d'éclairage. Dans l'exemple de la figure 2A, une voie de fixation, couplée au système par une lame de séparation LM4, est également prévue permettant de fixer l'attention du patient dont l'oeil est analysé. Elle comprend une image éclairée FIX et des moyens d'imagerie L4 assurant la conjugaison optique de l'image avec la rétine, ou réglé de manière à ce que l'image soit vue avec une légère défocalisation myopique pour stimuler la désaccommodation. Dans cet exemple, les voies d'analyse, d'éclairage, de fixation sont solidaires et positionnées sur une plate-forme PTF1 mobile le long de l'axe de mesure (z) par rapport aux moyens d'imagerie L1 afin d'assurer les conjugaisons optiques entre les différentes éléments du système en fonction de l'amétropie de l'oeil.

Les moyens d'analyse, par exemple un analyseur de type Shack-Hartmann, sont reliés aux moyens de traitement TRT, qui, de manière connue, établissent la cartographie en phase de l'onde émergente de l'oeil et calculent les aberrations. Une représentation de cette cartographie peut être affichée sur un écran SCR. Le système de correction comprend en outre le dispositif de correction MD qui intercepte le faisceau d'analyse FA et qui, commandé par l'unité de commande COM reliée aux moyens de traitement, permet de modifier localement la phase du front d'onde du faisceau d'analyse FA en fonction des valeurs des aberrations mesurées par les moyens de mesure MES_AB sur le faisceau de mesure FM. Le faisceau d'analyse FA suit un trajet optique similaire à celui du faisceau de mesure FM grâce à la lame de séparation LS ce qui assure une bonne cohérence entre les aberrations mesurées sur le faisceau FM et la correction subie par le faisceau FA. Le dispositif optique de correction MD est formé par exemple d'un miroir déformable constitué d'une surface réfléchissante pouvant être déformée par un ensemble d'actionneurs sur laquelle le faisceau d'analyse est incident, positionné sensiblement dans un plan conjugué de la pupille de l'oeil, chaque actionneur étant commandé par l'unité de commande pour corriger la phase locale du front d'onde du faisceau d'analyse. Il est également possible d'utiliser un modulateur spatial de lumière (ou SLM selon l'abréviation de l'expression anglo-saxonne « Spatial Light Modulator »), par exemple réalisé par une matrice de valves à cristaux liquides. Sur les figures 2A à 2C, une lame séparatrice LS positionnée sur la voie d'analyse envoie une partie du faisceau d'analyse FA après correction vers l'objectif et le détecteur d'imagerie rétinienne.

Cependant ces moyens ne sont pas suffisants pour compenser les aberrations résultant des mouvements oculaires dont la fréquence d'évolution est supérieure à la fréquence d'évolution des aberrations intrinsèques. Ces mouvements oculaires, comprenant des déplacements latéraux et/ou axiaux de la pupille et des mouvements de rotation de l'axe oculaire, faussent d'une part la correction des aberrations, et entraînent d'autre part des imprécisions sur la focalisation du faisceau d'éclairage sur la rétine. Ce dernier effet est particulièrement gênant pour la formation des images super résolues obtenues par combinaison d'images issues de la même zone de la rétine.

L'invention propose une méthode de correction des aberrations de l'oeil permettant de remédier aux inconvénients ci-dessus mentionnés et, plus généralement, d'améliorer la qualité d'un instrument ophtalmique fonctionnant avec un faisceau lumineux d'analyse. La méthode de correction des aberrations de l'oeil selon l'invention comprend en plus de la mesure des aberrations de l'oeil et de la correction de la phase du front d'onde du faisceau d'analysé en fonction des valeurs mesurées desdites aberrations, la mesure des mouvements de l'oeil réalisée indépendamment de ladite mesure des aberrations, et la modification de la correction de la phase du front d'onde du faisceau d'analyse en fonction de ladite mesure des mouvements de l'oeil. Le fait de procéder à une mesure indépendante des mouvements de l'oeil, c'est-à-dire en pratique réalisé par des moyens différents de ceux utilisés pour la mesure des aberrations, permet d'apporter au système une information supplémentaire, éventuellement à une fréquence plus rapide, et ainsi d'obtenir une correction des mouvements de l'oeil plus rapide, adaptée à l'évolution de ces mouvements.

La méthode s'applique aussi bien à un fonctionnement continu, dans lequel la mesure des aberrations est réalisée à une fréquence de récurrence donnée, qu'à un fonctionnement impulsionnel ou 'one shot', dans lequel on cherche à corriger le système à des instants donnés, par exemple dans le cas de tirs laser pour le traitement de la rétine. Dans le cas où la mesure des aberrations est réalisée en continu, à une fréquence donnée, la modification de la correction peut, grâce à l'invention, être réalisée à une fréquence de fonctionnement strictement supérieure à la fréquence de mesure des aberrations. Par exemple, la mesure des mouvements de l'oeil est réalisée elle-même en continu, à une fréquence strictement supérieure à la fréquence de mesure des aberrations, avantageusement multiple de ladite fréquence de mesure des aberrations, la fréquence de fonctionnement pouvant être égale à la fréquence de mesure des mouvements de l'oeil; ou même supérieure dans le cas par exemple où la mesure des mouvements de l'oeil est réalisée avec un décalage temporel par rapport à la mesure des aberrations. Typiquement, la fréquence de mesure des aberrations est comprise entre quelques Hertz et quelques dizaines de Hertz, et la fréquence de mesure des mouvements de l'oeil est de l'ordre d'une centaine de Hertz, ce qui permet en final une modification de la correction des aberrations à une vitesse suffisante pour prendre en compte les mouvements de l'oeil et améliorer ainsi la qualité de l'instrument. En fonctionnement impulsionnel, le processus de correction des aberrations de l'oeil comprend de la même façon une phase de mesure des aberrations optiques de l'oeil grâce à l'analyseur de front d'onde, une phase de mesure des mouvements de l'oeil, puis le calcul et l'application de la modification de la correction à l'élément de correction MD. L'exploitation du faisceau d'analyse FA est optimale à l'instant où l'élément de correction MD réalise la correction désirée. Lorsque les aberrations à corriger évoluent rapidement, plus le temps entre le début et la fin du processus de correction sera court, meilleur sera le résultat de l'exploitation du faisceau d'analyse. Dans ce cas, il sera intéressant de synchroniser les phases de mesure des aberrations et de mesures des mouvements oculaires de façon à réduire au maximum le temps de mesure global. Dans certaines applications, il est également possible de coupler des phases de fonctionnement continu et des phases de fonctionnement impulsionnel. Dans le cas de fonctionnement impulsionnel, on peut étendre la notion de fréquence à l'inverse de la durée de la mesure.

Selon l'invention, la mesure des mouvements de l'oeil peut comprendre l'estimation des déplacements latéraux et/ou axiaux de la pupille de l'oeil par rapport à une position prédéterminée. Dans le cas d'un instrument ophtalmique présentant un axe de visée devant être stabilisé par rapport à l'axe oculaire, la mesure des mouvements de l'oeil peut comprendre en outre l'estimation de la rotation relative de l'axe de l'oeil par rapport à une position prédéterminée. Par axe de visée, on entend la direction vers laquelle regarde ou vise l'instrument, ou la ligne qui joint le centre de la pupille d'entrée de l'instrument (qui peut être dans un plan confondu de celui de la pupille de l'oeil) au point que vise ou regarde l'instrument. La modification de la correction peut alors être faite également en fonction de l'estimation de la rotation de l'axe de l'oeil.

Selon une variante, lorsque la méthode selon l'invention est appliquée à un instrument d'imagerie rétinienne présentant un axe de visée devant être stabilisé par rapport à l'axe oculaire, la mesure des mouvements de l'oeil peut comprendre pour chaque prise d'image de la rétine l'estimation de la rotation relative de l'axe de l'oeil par rapport à une position prédéterminée, et la méthode peut inclure en outre une étape ultérieure de traitement desdites images permettant le recalage des images en fonction des estimations de rotation de l'axe de visée.

La figure 2A illustre un exemple de système de correction selon l'invention pour la mise en oeuvre de la méthode de correction ci-dessus décrite, permettant de compenser les effets des mouvements oculaires dans l'exemple d'un instrument d'imagerie rétinienne. Pour cela, il comprend un dispositif de mesure MES_OC des mouvements de l'oeil. Ce dispositif de mesure est distinct des moyens de mesure des aberrations MES_AB ce qui lui permet d'analyser les variations des mouvements oculaires à une fréquence de fonctionnement donnée rapide, typiquement de l'ordre de la centaine de Hz., et en tout état de cause au moins égale à la fréquence de mesure des aberrations de l'oeil. Le dispositif de mesure MES_OC, dont un exemple de réalisation est décrit en détail par la suite, est positionné sur l'axe de mesure z. Les moyens de séparation LM1 permettent de séparer la voie de mesure des mouvements oculaires des autres voies du système de correction, montées sur la plate-forme PTF1. Avantageusement, la plate-forme PTF1, les moyens d'imagerie L1 et le dispositif MES_OC sont positionnés sur une seconde plate-forme PTF2 mobile pour ajuster l'ensemble du système de correction au positionnement de la pupille de l'oeil.

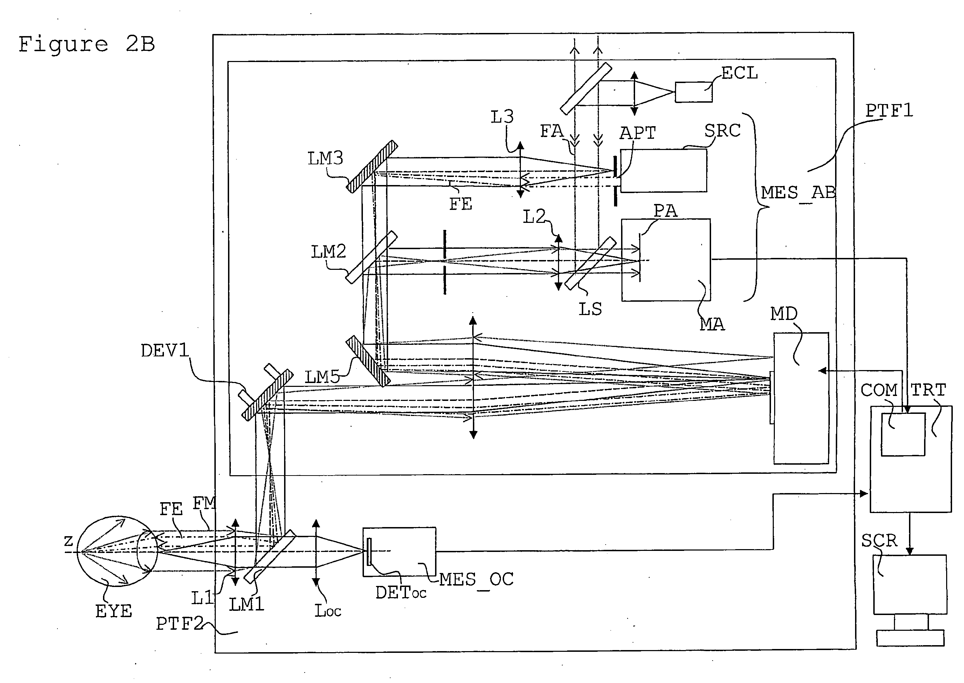

Le dispositif de mesure MES_OC permet d'estimer les déplacements de la pupille de l'oeil par rapport à une position prédéterminée. Les valeurs de déplacement sont envoyées à l'unité de commande COM afin de modifier à la fréquence de fonctionnement la correction de la phase du front d'onde du faisceau d'analyse. Selon l'invention, la modification de la correction peut être faite de plusieurs manières illustrées sur les figures 2A et 2B. Selon une première variante (figure 2A), des nouvelles valeurs de correction sont calculées par les moyens de traitement TRT de l'unité de commande du dispositif de correction en fonction des valeurs mesurées de déplacement de la pupille. Puis la modification de la correction de la phase du front d'onde est faite par application au dispositif de correction MD des nouvelles valeurs de correction calculées, à la fréquence de fonctionnement du dispositif de mesure MES_OC. Selon une autre variante (Figure 2B), il peut être prévu un élément optique DEV1 de déviation du faisceau d'analyse, relié à l'unité de commande, qui permet de stabiliser à la fréquence de fonctionnement la position de l'image de la pupille de l'oeil sur le dispositif de correction MD en fonction des valeurs de déplacement de la pupille. Avantageusement, l'élément optique DEV1 est positionné dans un plan proche d'un plan conjugué de la rétine de l'oeil, ce qui permet dans l'application d'imagerie rétinienne de ne pas perturber la formation de l'image de la rétine.

Dans une application de type imagerie rétinienne, ou dans tout autre application dans laquelle l'instrument ophtalmique présente un axe de visée que l'on souhaite stabiliser par rapport à l'axe oculaire, le dispositif de mesure MES_OC permet en outre d'estimer la rotation relative de l'axe de l'oeil par rapport à une position prédéterminée. Les valeurs de rotation sont envoyées à l'unité de commande afin de contrôler à la fréquence de fonctionnement du dispositif de mesure MES_OC, l'orientation de la ligne de visée de l'instrument. Comme précédemment, le contrôle de l'orientation de la ligne de visée peut être fait par application à la fréquence de fonctionnement de nouvelles valeurs de correction calculées par les moyens de traitement de l'unité de commande en fonction des valeurs de rotation mesurées de l'axe de l'oeil. Cela demande que le dispositif optique de correction MD, de type miroir déformable, ait une dynamique de fonctionnement suffisante.

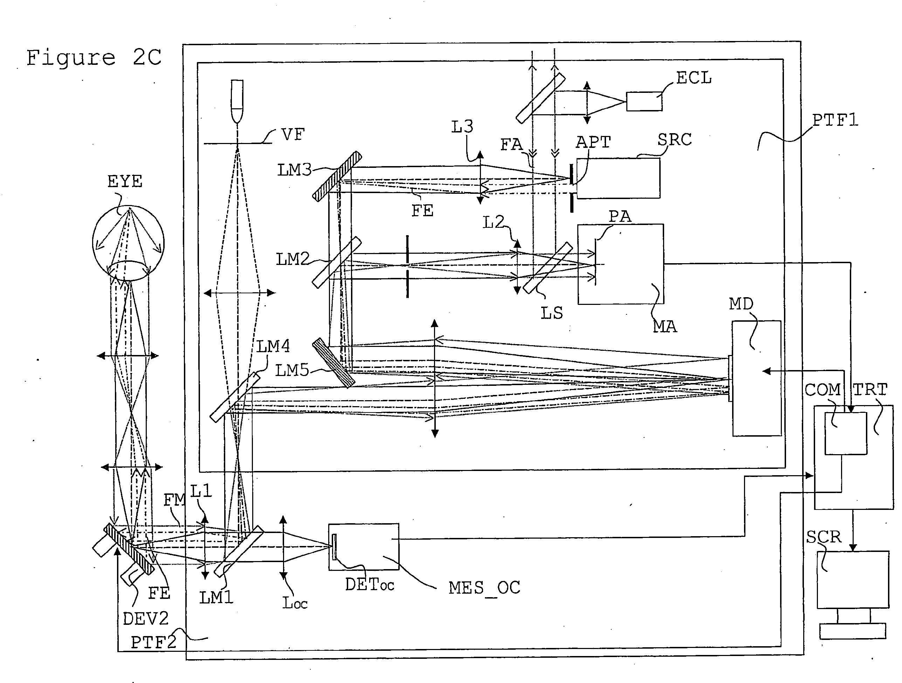

Selon une variante représentée sur la figure 2C, il est également possible de prévoir un élément optique DEV2 de déviation du faisceau d'analyse, relié à l'unité de commande, et permettant de contrôler à la fréquence de fonctionnement l'orientation de la ligne de visée en fonction des valeurs de rotation relative de l'axe de l'oeil. Dans ce cas, avantageusement, l'élément optique est positionné dans un plan proche d'un plan conjugué de la pupille de l'oeil, afin de ne pas perturber la correction des aberrations en modifiant la position de l'image de la pupille sur l'élément optique de correction.

Le système de correction selon l'invention s'applique également à un instrument de traitement optique dont un exemple de réalisation est montré sur la même figure 2A. On retrouve les différentes voies du système de correction précédemment décrites, à savoir voie d'éclairage, voie d'analyse, voie de fixation et voie de mesure des mouvements oculaires. A la différence de l'instrument d'imagerie rétinienne, l'instrument de traitement oculaire comprend une source laser (non représéntée spécifiquement sur la figure 2A mais qui pourrait prendre lieu et place de la source ECL d'éclairage de la rétine) pour l'émission d'un faisceau d'analyse FA destiné à être focalisé sur la rétine de l'oeil selon un axe de visée donné. Pour contrôler le diamètre et la forme de la tache laser formée sur la rétine, l'instrument peut être équipé d'un système de correction classique avec les moyens de mesure MES_AB des aberrations de l'oeil, le dispositif optique MD de correction de la phase du front d'onde du faisceau d'analyse et l'unité de commande COM tels que décrits précédemment. Le système de correction selon l'invention permet en outre d'adapter la correction en fonction des mouvements oculaires avec une fréquence rapide compatible de la fréquence d'évolution de ces mouvements, et d'améliorer la précision de la position de l'impact du faisceau laser sur la rétine grâce à une stabilisation de l'axe de visée du faisceau d'analyse FA par rapport à l'axe oculaire, dont les variations de l'orientation sont mesurées par le dispositif de mesure MES_OC.

La figue 3 montre selon un exemple comment le système de correction selon l'invention peut s'adapter tout aussi bien à un instrument ophtalmique de type instrument de simulation de la vision utilisé pour simuler à un patient l'image d'une mire qu'il pourrait voir avec une correction donnée. Un tel instrument comprend une voie de fixation semblable avec celle décrite précédemment comprenant une source lumineuse SF émettant un faisceau d'analyse FA et une image FIX éclairée par le faisceau d'analyse FA destinée à être vue par le patient. Il comprend comme précédemment des moyens de mesure MES_AB des aberrations de l'oeil, un dispositif optique MD de correction de la phase du front d'onde du faisceau d'analyse et une unité de commande COM. Dans ce cas, les moyens de traitement TRT de l'unité de commande calculent en fonction des aberrations de l'oeil mesurées, auxquelles sont ajoutés éventuellement les effets d'une correction de type verre correcteur, lentille de contact, etc., les corrections à appliquer à la phase du front d'onde du faisceau d'analyse par le dispositif optique de correction MD. Selon l'invention, un système de correction est prévu pour tenir compte dans le calcul des corrections à appliquer à la phase du front d'onde, des effets des mouvements oculaires. Comme précédemment, il comprend un dispositif de mesure des mouvements oculaires MES_OC permettant de modifier, à une fréquence compatible de celle des mouvements oculaires, les valeurs de correction de la phase du front d'onde du faisceau d'analyse en fonction des mouvements latéraux de la pupille. Là encore, la modification peut être faite par calcul ou grâce à un élément optique de déviation.

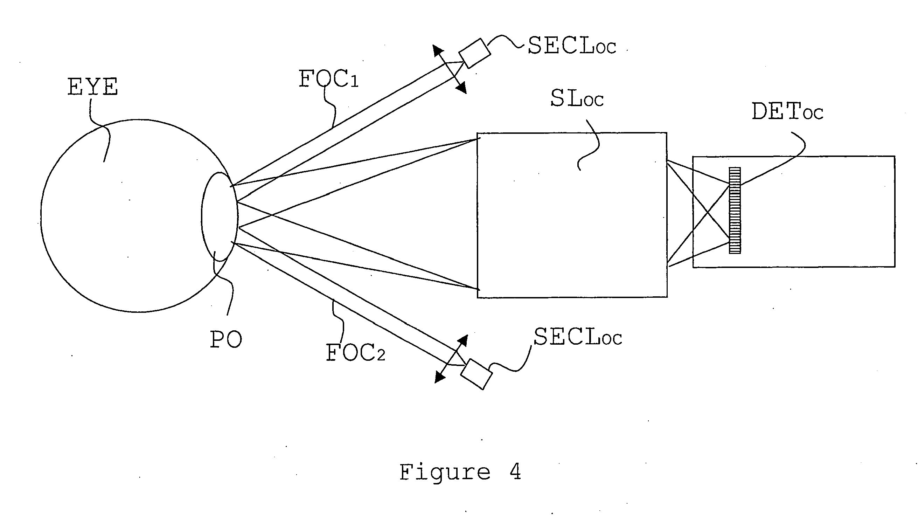

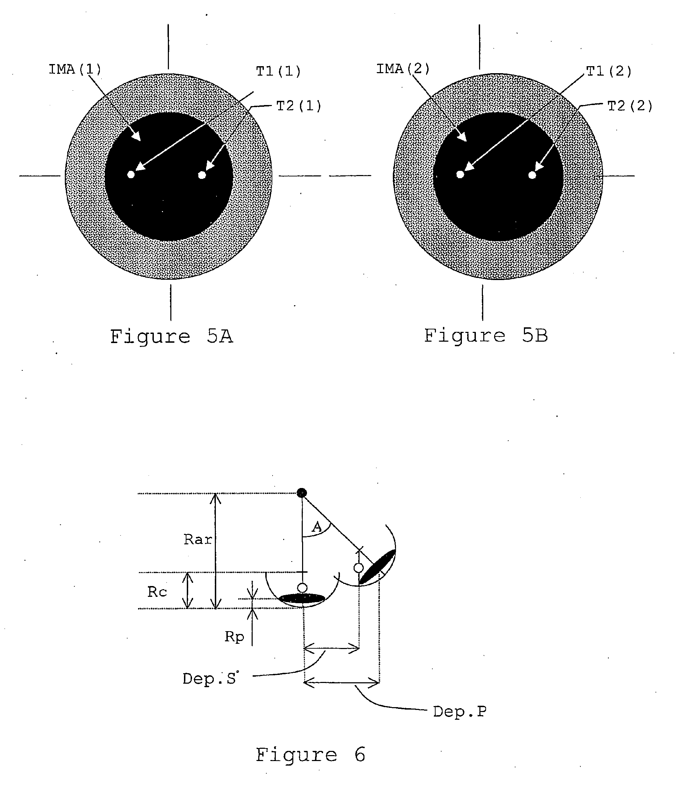

Nous décrivons maintenant un exemple de réalisation du dispositif de mesure des mouvements oculaires du système de correction selon l'invention à l'aide de l'exemple décrit sur la figure 4 et dont les figures 5A, 5B et 6 permettent de comprendre le fonctionnement. Le dispositif décrit sur la figure 4 est un dispositif de type dispositif optique. D'autres dispositifs sont envisageables, comme par exemple un dispositif basé sur une détection d'un champ magnétique généré par des lentilles de contact munies de bobines d'inductance. Mais un dispositif de type optique, sans contact, est préféré pour la mise en oeuvre de l'invention. Le dispositif de mesure des mouvements de l'oeil MES_OC comprend selon cet exemple un détecteur matriciel $DET_{OC}$, un objectif $SL_{OC}$ formant l'image IMA de la pupille PO de l'oeil EYE sur le détecteur, dés moyens d'éclairage $SECL_{OC}$ émettant chacune un faisceau sensiblement collimaté $F_{OC1}$, $F_{OC2}$ incident sur la pupille de l'oeil PO et formant deux taches lumineuses sur ladite pupille, des moyens de traitement (non représentés sur la figure 4), qui peuvent être les moyens de traitement TRT des figures 2A à 2C, permettant de déterminer les variations des mouvements de l'oeil à partir des positions relatives des images T1, T2 formées par l'objectif $SL_{OC}$ desdites taches par rapport à l'image IMA de la pupille.

La figure 5A représente l'image sur le détecteur $DET_{OC}$ de la pupille de l'oeil IMA(1) et les images T1(1), T2(1) des deux taches formées par les moyens d'éclairage sur la pupille dans une première position de l'oeil du patient. Dans le cas d'un déplacement de la tête correspondant à un mouvement de translation pure, sans rotation (sans variation de l'axe de visée de l'oeil), compte tenu que les faisceaux d'éclairage sont collimatées, l'image des sources d'éclairage par la cornée (équivalent à un miroir convexe) est toujours au même endroit dans le référentiel de la cornée. Autrement dit, une translation pure de l'oeil d'une valeur donnée (sans variation de l'axe de visée de l'oeil) entraîne sur le détecteur une translation de la même valeur de l'image de la pupille et des images des sources par la cornée (connues sous le nom d'images de Purkinje). Sur la figure 5B correspondant à une deuxième position de l'oeil, on observe ainsi un déplacement identique de l'image de la pupille IMA(2) et des images de Purkinje T2(1) et T2(2) lors d'une translation de la tête du patient.

Sur la figure 6 est illustré le cas d'une variation de l'orientation de l'axe de visée d'un angle A par rapport à l'axe de rotation de l'oeil (sans mouvement de la tête). On note Rar la distance qui sépare le sommet de la cornée de l'axe de rotation de l'oeil, Rc le rayon de courbure de la cornée et Rp la distance séparant le sommet de la cornée et l'image de la pupille par la cornée. Les relations suivantes sont vérifiées :

$$Dep.P(rot) = (Rar - Rp)*sin(A)$$

$$\texttt{Dep.S(rot) = (Rar - Rc)*sin(A)}$$

Où Dep.P(rot) et Dep.S(rot) sont respectivement les déplacements dus à un mouvement de rotation pur, sur le détecteur $DET_{OC}$, de l'image de la pupille et de la source lumineuse.

Dans le cas d'un mouvement à la fois en translation et en rotation de la pupille de l'oeil, on observe sur le détecteur un déplacement de l'image de la pupille d'une valeur Dep.P(trans+rot) ainsi qu'un déplacement des images des sources lumineuses d'un déplacement Dep.S(trans+rot). La valeur de déplacement de l'image de la pupille Dep.P est traitée par les moyens de traitement TRT pour commander le dispositif de correction MD en vue de modifier les corrections des aberrations de l'oeil en fonction des déplacements latéraux de la pupille. On obtient la valeur de variation de l'axe de visée de l'oeil :

$$\texttt{Dep.P(trans+rot) - Dep.S(trans+rot) = (Rc-Rp) * sin(A)}$$

Rc est facilement mesurable par des mesures annexes (par exemple un kératomètre, ou par un topographe cornéen) et Rp peut être mesurée grâce à un instrument de tomographie. Ainsi la relation donnant la valeur de l'angle A est la suivante :

$$\texttt{A = asin ((Dep.P(trans+rot) - Dep.S(trans+rot))/(Rc-Rp))}$$

Cette valeur est envoyée si nécessaire à l'unité de commande COM afin d'obtenir la stabilisation de l'axe de visée du faisceau d'analyse par rapport à l'axe oculaire.

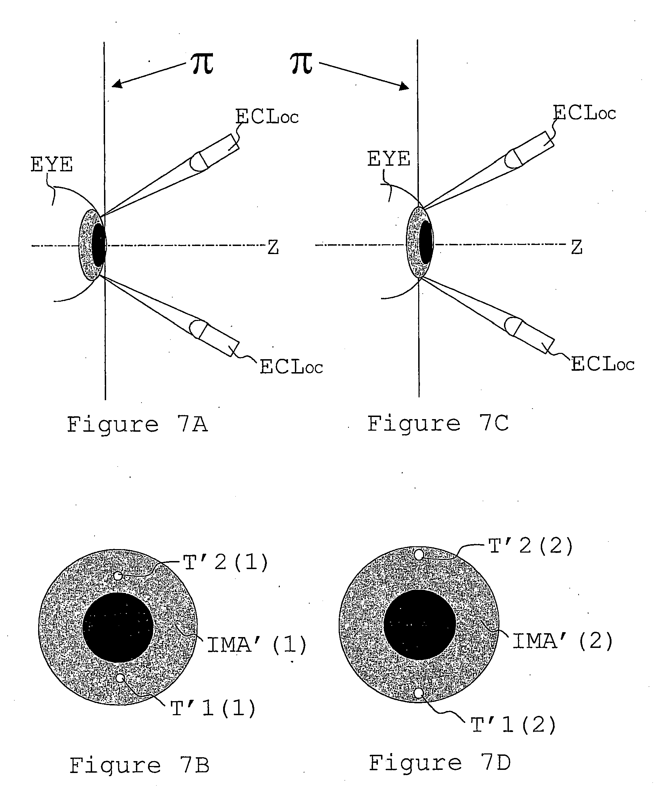

Les figures 7A à 7D illustrent le principe de la mesure des déplacements axiaux de la pupille de l'oeil grâce à un dispositif du type de celui décrit sur la figure 4 mais dans lequel le système d'éclairage est différent. Pour la mesure des déplacements axiaux, le système d'éclairage $ECL_{OC}$ comprend des moyens de formation de deux sources lumineuses ponctuelles ou quasi ponctuelles, selon des axes optiques distincts l'un de l'autre et distincts également de l'axe de visée z de l'instrument optique, par exemple l'instrument d'imagerie rétinienne, sur une partie diffusante de l'oeil, l'iris ou le blanc de l'oeil. Les images de ces sources sont observés au moyen d'un système d'imagerie et d'un détecteur du type de ceux décrits sur la figure 4. Le système d'éclairage peut utiliser les mêmes moyens que celui décrit à partir de la figure 4 mais réglés différemment, puisque dans le cas de mesure de déplacements latéraux, le système d'éclairage envoie deux faisceaux sensiblement collimatés. L'espace entre les deux images des sources formées sur le détecteur est proportionnel à la distance qui sépare la pupille de l'oeil d'un plan π donné, par exemple le plan de meilleure mise au point. Ainsi la figure 7A représente l'oeil EYE du patient dans une première position par rapport au plan π et la figure 7B l'image IMA' (1) de la pupille de l'oeil EYE dans cette première position avec l'image correspondante des sources ponctuelles (T'1(1), T'2(1)) sur le détecteur de mesure des mouvements oculaires. La figure 7C représente l'oeil EYE du patient dans une seconde position par rapport au plan π et la figure 7B l'image IMA'(1) de la pupille de l'oeil EYE dans cette première position avec l'image correspondante des sources ponctuelles (T'1(2), T'2(2)). La mesure de l'espace entre les deux images des sources permet de déduire le déplacement axial par rapport au plan π de référence.

**Revendications**

1. Méthode de correction des aberrations de l'oeil appliquée à un instrument ophtalmique fonctionnant avec un faisceau lumineux d'analyse (FA), comprenant:

   - la mesure des aberrations de l'oeil susceptibles de perturber ledit faisceau d'analyse,
   - la correction de la phase du front d'onde dudit faisceau d'analyse en fonction des valeurs mesurées desdites aberrations,
   **caractérisée en ce qu'**elle comprend en outre:
   - la mesure des mouvements de l'oeil réalisée indépendamment de ladite mesure des aberrations,
   - la modification de la correction de la phase du front d'onde du faisceau d'analyse en fonction de ladite mesure des mouvements de l'oeil.

2. Méthode de correction selon la revendication 1, dans laquelle la mesure des aberrations se faisant à une fréquence donnée, la modification de la correction est réalisée à une fréquence de fonctionnement, strictement supérieure à ladite fréquence de mesure des aberrations.

3. Méthode de correction selon la revendication 2, dans laquelle ladite mesure des mouvements de l'oeil est réalisée à une fréquence strictement supérieure à ladite fréquence de mesure des aberrations, multiple de ladite fréquence de mesure des aberrations, la fréquence de fonctionnement étant supérieure ou égale à la fréquence de mesure des mouvements de l'oeil.

4. Méthode de correction selon l'une des revendications 2 ou 3, dans laquelle la fréquence de mesure des aberrations est comprise entre quelques Hertz et quelques dizaines de Hertz, et la fréquence de mesure des mouvements de l'oeil est de l'ordre d'une centaine de Hertz.

5. Méthode de correction selon l'une des revendications précédentes, dans laquelle la mesure des mouvements de l'oeil comprend une mesure desdits mouvements réalisée avec un décalage temporel par rapport à ladite mesure des aberrations.

6. Méthode selon l'une des revendications précédentes, dans laquelle l'instrument ophtalmique présentant un axe de visée devant être stabilisé par rapport à l'axe oculaire, la mesure des mouvements de l'oeil comprend l'estimation de la rotation relative de l'axe de l'oeil par rapport à une position prédéterminée, et la modification de la correction est faite en fonction de ladite estimation de la rotation de l'axe de l'oeil.

7. Méthode selon l'une des revendications précédentes, dans laquelle ladite mesure des mouvements de l'oeil comprend notamment l'estimation des déplacements latéraux et/ou axiaux de la pupille de l'oeil par rapport à une position prédéterminée.

8. Méthode selon l'une des revendications 6 ou 7 appliquée à un instrument d'imagerie rétinienne présentant un axe de visée devant être stabilisé par rapport à l'axe oculaire, dans laquelle la mesure des mouvements de l'oeil comprend pour chaque prise d'image de la rétine l'estimation de la rotation relative de l'axe de l'oeil par rapport à une position prédéterminée, et la méthode comprend en outre une étape ultérieure de traitement desdites images permettant le recalage des images en fonction des estimations de rotation de l'axe de visée.

9. Système de correction des aberrations de l'oeil destiné à être intégré dans un instrument ophtalmique fonctionnant avec un faisceau lumineux d'analyse (FA), comprenant:

    - des moyens de mesure (MES_AB) des aberrations de l'oeil susceptibles de perturber ledit faisceau d'analyse,
    - un dispositif optique (MD) de correction de la phase du front d'onde dudit faisceau d'analyse,
    - une unité de commande (COM) du dispositif de correction avec des moyens de traitement (TRT) reliés aux moyens de mesure et calculant les valeurs de correction à appliquer au dispositif de correction en fonction des valeurs mesurées des aberrations,
    le système de correction étant **caractérisé en ce qu'**il comprend
    - un dispositif de mesure (MES_OC) des mouvements de l'oeil distinct desdits moyens de mesure des aberrations, lesdites valeurs de mesures des mouvements de l'oeil étant envoyées à l'unité de commande afin de modifier la correction de la phase du front d'onde du faisceau d'analyse.

10. Système de correction selon la revendication 9, dans lequel le dispositif de mesure des mouvements de l'oeil permettant notamment d'estimer les déplacements latéraux et/ou axiaux de la pupille de l'oeil par rapport à une position prédéterminée, ladite modification de la phase du front d'onde est faite par application de nouvelles valeurs de correction calculées par les moyens de traitement en fonction desdites valeurs de déplacement de la pupille.

11. Système de correction selon la revendication 9, dans lequel le dispositif de mesure des mouvements de l'oeil permettant notamment d'estimer les déplacements latéraux et/ou axiaux de la pupille de l'oeil par rapport à une position prédéterminée, la système comprend en outre un premier élément optique de déviation du faisceau d'analyse, positionné dans un plan proche d'un plan conjugué de la rétine de l'oeil, relié à l'unité de commande, et permettant de stabiliser la position de l'image de la pupille de l'oeil sur le dispositif de correction en fonction desdites valeurs de déplacement de la pupille.

12. Système de correction selon l'une des revendications 9 à 11 destiné à un instrument ophtalmique présentant un axe de visée devant être asservi par rapport à l'axe oculaire, dans lequel le dispositif de mesure des mouvements de l'oeil permet l'estimation de la rotation relative de l'axe de l'oeil par rapport à une position prédéterminée, lesdites valeurs de rotation étant envoyées à l'unité de commande afin de modifier la correction de la phase du front d'onde du faisceau d'analyse.

13. Système de correction selon la revendication 12, dans lequel la modification de la correction de la phase du front d'onde du faisceau d'analyse est faite par application de nouvelles valeurs de correction calculées par l'unité de commande du dispositif de correction en fonction des valeurs de rotation relative de l'axe de l'oeil.

14. Système de correction selon la revendication 12, comprenant en outre un élément optique de déviation du faisceau d'analyse, positionné dans un plan proche d'un plan conjugué de la pupille de l'oeil, relié à l'unité de commande, et permettant de contrôler à ladite fréquence de fonctionnement l'orientation de la ligne de visée en fonction desdites valeurs de rotation relative de l'axe de l'oeil.

15. Système de correction selon l'une des revendications 9 à 14, dans lequel le dispositif de mesure (MES_OC) des mouvements de l'oeil comprend un détecteur matriciel (DET$_{OC}$), un objectif (L$_{OC}$) formant l'image (IMA) de la pupille de l'oeil sur le détecteur, des moyens d'éclairage permettant de former deux faisceaux collimatés incidents sur la pupille de l'oeil et formant deux taches lumineuses (T1, T2) sur ladite pupille, des moyens de traitement permettant de déterminer les variations des mouvements de l'oeil à partir des positions relatives des taches par rapport à image de la pupille.

16. Système de correction selon l'une des revendications 9 à 15, dans lequel le dispositif optique de correction est un miroir déformable constitué d'une surface réfléchissante pouvant être déformée par un ensemble d'actionneurs sur lequel ledit faisceau d'analyse est incident, positionné sensiblement dans un plan conjugué de la pupille de l'oeil, chaque actionneur étant commandé par l'unité de commande pour corriger la phase locale du front d'onde du faisceau d'analyse.

17. Système de correction selon l'une des revendications 9 à 16, dans lequel les moyens de mesure (MES_AB) des aberrations de l'oeil comprennent un système d'éclairage avec notamment une source d'émission (SRC) d'un faisceau d'éclairage (FE) pour former par rétrodiffusion sur la rétine une source lumineuse secondaire, ladite source secondaire émettant un faisceau de mesure (FM) émergent de l'oeil, et des moyens d'analyse de la phase du front d'onde dudit faisceau de mesure.

18. Système de correction selon la revendication 17, dans lequel les moyens d'analyse comprennent un analyseur de type Shack-Hartmann avec notamment un détecteur matriciel, une matrice de microlentilles recevant ledit faisceau de mesure et formant sur le détecteur matriciel un ensemble de taches lumineuses, des moyens de traitement desdites taches lumineuses pour la détermination des aberrations.

19. Instrument d'imagerie rétinienne comprenant un système d'éclairage de la rétine émettant un faisceau d'éclairage d'axe donné, un système d'imagerie et de détection recevant un faisceau d'analyse émergent de l'oeil et réalisant la fonction de détection de l'image de la rétine sur le détecteur dudit système d'imagerie et de détection, **caractérisé en ce qu'**il comprend un système de correction des aberrations de l'oeil selon l'une des revendications 12 à 18, permettant la correction de la phase du front d'onde du faisceau d'analyse notamment en fonction de l'estimation de la rotation relative de l'axe de l'oeil par rapport à une position prédéterminée.

20. Instrument de traitement optique par laser sur la rétine comprenant une source laser pour l'émission d'un faisceau d'analyse destiné à être focalisé sur la rétine de l'oeil selon un axe de visée donné, **caractérisé en ce qu'**il comprend un système de correction des aberrations de l'oeil selon l'une des revendications 12 à 18, permettant la correction de la phase du front d'onde du faisceau d'analyse notamment en fonction de l'estimation de la rotation relative de l'axe de l'oeil par rapport à une position prédéterminée.

21. Instrument de simulation de la vision d'un patient comprenant un système de fixation avec une source lumineuse émettant un faisceau d'analyse et une mire éclairée par le faisceau d'analyse et destinée à être vue par le patient, **caractérisé en ce qu'**il comprend un système de correction des aberrations de l'oeil selon l'une des revendications 9 à 18, permettant la correction de la phase du front d'onde du faisceau d'analyse en fonction des mouvements de l'oeil.

**Claims**

1.  Method for correcting aberrations of the eye applied to an ophthalmic instrument operating with an analysis light beam (FA), comprising:

    - measurement of aberrations of the eye capable of interfering with said analysis beam,
    - correction of the phase of the wave front of said analysis beam as a function of the measured values of said aberrations, **characterized in that** it also comprises:
    - measurement of eye movements carried out independently of said measurement of aberrations,
    - modification of the correction of the phase of the wave front of the analysis beam as a function of said measurement of eye movements.

2.  Correction method according to claim 1, in which the aberration measurement being carried out at a given frequency, the modification of the correction is carried out at an operating frequency strictly greater than said aberration measurement frequency.

3.  Correction method according to claim 2, in which said measurement of eye movements is carried out at a frequency strictly greater than said aberration measurement frequency, and that is a multiple of said aberration measurement frequency, the operating frequency being greater than or equal to the eye movement measurement frequency.

4.  Correction method according to one of claims 2 or 3, in which the aberration measurement frequency is comprised between a few Hertz and a few tens of Hertz, and the eye movement measurement frequency is of the order of a hundred Hertz.

5.  Correction method according to one of the preceding claims, in which the measurement of eye movements comprises a measurement of said movements carried out with a time shift with respect to said measurement of aberrations.

6.  Method according to one of the preceding claims, in which the ophthalmic instrument having a sight axis which must be stabilized with respect to the ocular axis, the measurement of eye movements comprises the estimation of the relative rotation of the axis of the eye with respect to a predetermined position, and the modification of the correction is carried out as a function of said estimation of the rotation of the axis of the eye.

7.  Method according to one of the preceding claims, in which said measurement of eye movements comprises in particular the estimation of the lateral and/or axial displacements of the pupil of the eye with respect to a predetermined position.

8.  Method according to one of claims 6 or 7 applied to a retinal imaging instrument having a sight axis which must be stabilized with respect to the ocular axis, in which the measurement of eye movements comprises for each image of the retina taken, the estimation of the relative rotation of the axis of the eye with respect to a predetermined position, and the method can also comprise a subsequent stage of processing of said images allowing the adjustment of the images as a function of the estimations of rotation of the sight axis.

9.  System for correcting aberrations of the eye intended to be integrated into an ophthalmic instrument operating with an analysis light beam (FA), comprising:

    - means (MES_AB) for measuring aberrations of the eye capable of interfering with said analysis beam,
    - an optical device (MD) for correcting the phase of the wave front of said analysis beam,
    - a control unit (COM) for the correction device with processing means (TRT) connected to the measurement means, and calculating the correction values to be applied to the correction device as a function of the measured values of the aberrations,
    the correction system being **characterized in that** it comprises
    - a device (MES_OC) for measuring eye movements which is separate from said means for measuring the aberrations, said eye movement measurement values being sent to the control unit in order to modify the correction of the phase of the wave front of the analysis beam.

10. Correction system according to claim 9, in which the device for measuring eye movements makes it possible in particular to estimate the lateral and/or axial displacements of the pupil of the eye with respect to a predetermined position, said modification of the phase of the wave front is carried out by applying new correction values calculated by the processing means as a function of said displacement values of the pupil.

11. Correction system according to claim 9 in which, the device for measuring eye movements makes it possible in particular to estimate the lateral and/or axial displacements of the pupil of the eye with respect to a predetermined position, the system also comprises a first optical element for deflecting the analysis beam, positioned in a plane close to a conjugated plane of the retina of the eye, connected to the control unit, and making it possible to stabilize the position of the image of the pupil of the eye on the correction device as a function of said displacement values of the pupil.

12. Correction system according to one of claims 9 to 11 intended for an ophthalmic instrument having a sight axis which must be controlled with respect to the ocular axis, in which the device for measuring eye movements allows the estimation of the relative rotation of the axis of the eye with respect to a predetermined position, said rotation values being sent to the control unit in order to modify the correction of the phase of the wave front of the analysis beam.

13. Correction system according to claim 12, in which the modification of the correction of the phase of the wave front of the analysis beam is carried out by applying new correction values calculated by the control unit of the correction device as a function of the values of relative rotation of the axis of the eye.

14. Correction system according to claim 12, also comprising an optical element for deflecting the analysis beam, positioned in a plane close to a conjugated plane of the pupil of the eye, connected to the control unit, and making it possible to control, at said operating frequency, the orientation of the line of sight as a function of said relative rotation values of the axis of the eye.

15. Correction system according to one of claims 9 to 14, in which the device (MES_OC) for measuring eye movements comprises an array detector ($DET_{OC}$), an objective ($L_{OC}$) forming the image (IMA) of the pupil of the eye on the detector, illumination means making it possible to form two collimated beams incident on the pupil of the eye and forming two light spots (T1, T2) on said pupil, processing means making it possible to determine variations in the eye movements from the relative positions of the spots with respect to the image of the pupil.

16. Correction system according to one of claims 9 to 15, in which the optical correction device is a deformable mirror constituted by a reflective surface which can be deformed by a set of actuators on which said analysis beam is incident, substantially positioned in a conjugated plane of the pupil of the eye, each actuator being controlled by the control unit in order to correct the local phase of the wave front of the analysis beam.

17. Correction system according to one of claims 9 to 16, in which the means (MES_AB) for measuring aberrations of the eye comprise an illumination system with in particular a source (SRC) for emitting an illumination beam (FE) in order to form a secondary light source on the retina by backscattering, said secondary source emitting a measurement beam (FM) emerging from the eye, and means for analyzing the phase of the wave front of said measurement beam.

18. Correction system according to claim 17, in which the analysis means comprise a Shack-Hartmann type analyzer with in particular an array detector, an array of microlenses receiving said measurement beam and forming on the array detector a set of light spots, and means for processing said light spots for determination of the aberrations.

19. Retinal imaging instrument comprising a retina illumination system emitting an illumination beam of given axis, an imaging and detection system receiving an analysis beam emerging from the eye and carrying out the function of detection of the image of the retina on the detector of said imaging and detection system, **characterized in that** it comprises a system for correcting aberrations of the eye according to one of claims 12 to 18, allowing the correction of the phase of the wave front of the analysis beam in particular as a function of the estimation of the relative rotation of the axis of the eye with respect to a predetermined position.

20. Instrument for optical laser treatment on the retina comprising a laser source for the emission of an analysis beam intended to be focussed on the retina of the eye along a given sight axis, **characterized in that** it comprises a system for correcting aberrations of the eye according to one of claims 12 to 18, allowing the correction of the phase of the wave front of the analysis beam in particular as a function of the estimation of the relative rotation of the axis of the eye with respect to a predetermined position.

**21.** Instrument for simulation of the vision of a patient comprising a fixation system with a light source emitting an analysis beam and a target illuminated by the analysis beam and intended to be seen by the patient, **characterized in that** it comprises a system for correcting aberrations of the eye according to one of claims 9 to 18, allowing the correction of the phase of the wave front of the analysis beam as a function of movements of the eye .

## Patentansprüche

**1.** Verfahren zur Korrektur der Aberrationen des Auges, an einem mit einem Analyselichtstrahl (FA) funktionierenden ophtalmischen Instrument angewandt, enthaltend:

- die Messung der den genannten Analysestrahl möglicherweise störenden Aberrationen des Auges und
- die Korrektur der Wellenfrontphase des genannten Analysestrahls entsprechend der Messwerte der genannten Aberrationen,
**dadurch gekennzeichnet, dass** es außerdem:
- die in Unabhängigkeit von der genannten Messung der Aberrationen durchgeführte Messung der Augenbewegungen und
- die Änderung der Korrektur der Wellenfrontphase des Analysestrahls entsprechend der genannten Messung der Bewegungen des Auges
umfasst.

**2.** Korrekturverfahren nach Anspruch 1, bei dem die Messung der Aberrationen zu einer gegebenen Frequenz erfolgt, wobei die Änderung der Korrektur zu einer Leistungsfrequenz durchgeführt wird, die höher als die genannte Messfrequenz der Aberrationen ist.

**3.** Korrekturverfahren nach Anspruch 2, bei dem die genannte Messung der Bewegungen des Auges zu einer Frequenz durchgeführt wird, die höher als die genannte Messfrequenz der Aberrationen ist und ein Vielfaches der genannten Messfrequenz der Aberrationen beträgt, wobei die Leistungsfrequenz höher als die Messfrequenz der Bewegungen des Auges oder gleich dieser Frequenz ist.

**4.** Korrekturverfahren nach Anspruch 2 oder 3, bei dem die Messfrequenz der Aberrationen zwischen einigen Hertz und einigen Dutzen Hertz liegt und die Messfrequenz der Bewegungen des Auges über Hundert Hertz beträgt.

**5.** Korrekturverfahren nach einem der vorhergehenden Ansprüche, bei dem die Messung der Bewegungen des Auges eine Messung der genannten Bewegungen umfasst, die im Vergleich zu der genannten Messung der Aberrationen zeitlich abweichend durchgeführt wird.

**6.** Verfahren nach einem der vorhergehenden Ansprüche, bei dem das ophtalmische Instrument eine entgegen der Augenachse zu stabilisierende Zielachse aufweist, wobei die Messung der Bewegungen des Auges dementsprechend die Schätzung der relativen Rotation der Augenachse entgegen einer vorbestimmten Lage umfasst und wobei die Korrektionsänderung anhand der vorher genannten Schätzung der Rotation der Augenachse erfolgt.

**7.** Verfahren nach einem der vorhergehenden Ansprüche, bei dem die genannte Messung der Bewegungen des Auges insbesondere die Schätzung der seitlichen und/oder achsialen Bewegungen der Augenpupille entgegen einer vorbestimmten Lage umfasst.

**8.** Verfahren nach einem der Ansprüche 6 oder 7, angewandt an einem eine gegenüber der Augenachse zu stabilisierende Zielachse aufweisenden Instrument für Netzhautbilder, bei dem die Messung der Bewegungen des Auges für jede Bildaufnahme durch die Netzhaut die Schätzung der relativen Rotation der Augenachse entgegen einer vorbestimmten Lage umfasst, und das Verfahren umfasst außerdem einen weiteren Schritt zur Verarbeitung der genannten Bilder, wobei die Registrierung der Bilder entsprechend den Schätzungen der Rotation der Zielachse ermöglicht wird.

**9.** System zur Korrektur der Aberrationen des Auges, für ein mit einem Analyselichtstrahl (FA) funktionierendes, ophtalmisches Instrument bestimmt, enthaltend:

- Messmittel (MES_AB) der möglicherweise den genannten Analysestrahl störenden Aberrationen des Auges,
- eine optische Vorrichtung (MD) zur Korrektur der Phase der Wellenfront des genannten Analysestrahls,

- eine Steuereinheit (COM) für die Korrektionsvorrichtung mit Verarbeitungsmitteln (TRT), wobei diese mit den Messmitteln verbunden sind und die an der Korrektionsvorrichtung entsprechend den Messwerten der Aberrationen anzuwendenden Korrektionswerte berechnen,
wobei das Korrektionssystem **dadurch gekennzeichnet ist, dass** es

- eine Messvorrichtung (MES_OC) der Bewegungen des Auges von den genannten Messmitteln der Aberrationen abweichend umfasst, wobei die genannten Messwerte der Bewegungen des Auges an die Steuereinheit zur Änderung der Korrektur der Wellenfrontphase des Analysestrahls gesendet werden.

10. Korrektionssystem nach Anspruch 9, bei dem die Messeinrichtung für die Bewegungen des Auges es insbesondere ermöglicht, die seitlichen und/oder achsialen Bewegungen der Augenpupille entgegen einer vorbestimmten Lage zu schätzen, wobei die genannte Änderung der Phase der Wellenfront durch die Anwendung der neuen, durch die Verarbeitungsmittel entsprechend den genannten Bewegungswerten der Pupille berechneten Korrektionswerte erfolgt.

11. Korrektionssystem nach Anspruch 9, bei dem die Messeinrichtung für die Bewegungen des Auges insbesondere die Schätzung der seitlichen und/oder achsialen Bewegungen der Augenpupille entgegen einer vorbestimmten Lage ermöglicht, wobei das System außerdem ein erstes optisches Element zur Abweichung des Analysestrahls umfasst, das in einer Ebene neben einer gekoppelten Ebene der Netzhaut des Auges angeordnet ist, mit der Steuereinheit verbunden ist und die Stabilisierung der Bildlage der Augenpupille auf der Korrektionsvorrichtung entsprechend den genannten Bewegungswerten der Pupille ermöglicht.

12. Korrektionssystem nach einem der Ansprüche 9-11 für ein ophtalmisches, eine entsprechend der Augenachse zu justizierende Zielachse aufweisendes Instrument bestimmt, bei dem die Messeinrichtung für die Bewegungen des Auges die Schätzung der relativen Rotation der Augenachse entgegen einer vorbestimmten Lage ermöglicht, wobei die vorher genannten Rotationswerte zur Änderung der Korrektur der Wellenfrontphase des Analysenstrahls an die Steuereinheit gesendet werden.

13. Korrektionssystem nach Anspruch 12, wobei die Änderung der Korrektur der Wellenfrontphase des Analysenstrahls durch Anwendung neuer, durch die Steuereinheit der Korrektionsvorrichtung entsprechend den relativen Rotationswerten der Augenachse berechneter Korrektionswerte erfolgt.

14. Korrektionssystem nach Anspruch 12, außerdem ein optisches Element zur Abweichung des Analysenstrahls umfassend, das in einer Ebene nahe einer gekoppelten Ebene der Augenpupille angeordnet ist, mit der Steuereinheit verbunden ist und die Überwachung der Orientierung der Ziellinie entsprechend den vorher genannten relativen Rotationswerten der Augenachse zu der vorher genannten Leistungsfrequenz ermöglicht.

15. Korrektionssystem nach einem der Ansprüche 9-14, bei dem die Messeinrichtung (MES_OC) für die Bewegungen des Auges einen Matrixsensor (DET$_{OC}$), ein das Bild (IMA) der Augenpupille auf dem Sensor erzeugende Objektiv (L$_{OC}$), Beleuchtungsmittel zur Erzeugung zweier begrenzten, einfallenden Strahlen auf der Pupille und zwei Lichtflecken (T1, T2) auf der genannten Pupille erzeugend, sowie Verarbeitungsmittel zur Ermittlung der Veränderungen der Augenbewegungen anhand der relativen Lagen der Lichtflecken entgegen dem Pupillenbild umfasst.

16. Korrektionssystem nach einem der Ansprüche 9-15, bei dem die optische Korrektionsvorrichtung ein verformbarer Spiegel ist, der aus einer spiegelnden, durch ein Gefüge von Stellantrieben verformbaren Fläche besteht, auf welche der genannte Analysenstrahl einfällt, und der in einer gekoppelten Ebene der Augenpupille angeordnet ist, wobei jeder Stellantrieb durch die Steuereinheit zur Korrektur der lokalen Phase der Wellenfront des Analysenstrahls gesteuert wird.

17. Korrektionssystem nach einem der Ansprüche 9-16, bei dem die Messmittel (MES_AB) der Aberrationen des Auges ein Beleuchtungssystem mit insbesondere einer Ausgabequelle (SRC) eines Beleuchtungsstrahls (FE) zur Erzeugung einer Zweitlichtquelle auf der Netzhaut durch Rückstreuung umfassen, wobei die genannte Zweitlichtquelle einen von dem Augen ausfallenden Bemessungsstrahl (FM) ausgibt, sowie Analysemittel der Wellenfrontphase des genannten Bemessungsstrahls.

18. Korrektionssystem nach Anspruch 17, bei dem die Analysemittel einen Shack-Hartmann-Sensor mit insbesondere einem Matrixsensor, eine den genannten Bemessungsstrahl empfängende und auf dem Matrixsensor ein Gefüge von Lichtflecken erzeugende Mikrolinsenmatrix, sowie Mittel zur Verarbeitung der genannten Lichtflecken zur Ermittlung der Aberrationen umfassen.

19. Instrument für Netzhautbilder mit einem einen Beleuchtungsstrahl mit bestimmter Achse ausgebendes System zur Beleuchtung der Netzhaut, mit einer Abbildungs- und Überwachungsvorrichtung, einen von dem Augen ausfallenden Analysestrahl empfängend und die Rolle zur Überwachung des Netzhautbildes auf dem Sensor der genannten Abbildungs- und Überwachungsvorrichtung ausführend, **dadurch gekennzeichnet, dass** es ein System zur Korrektur der Aberrationen des Auges nach einem der Ansprüche 12-18 umfasst, wobei die Korrektur der Wellenfrontphase des Analysestrahls insbesondere entsprechend der Schätzung der relativen Rotation der Augenachse entgegen einer vorbestimmten Lage ermöglicht wird.

20. Instrument für optische Behandlung durch Laserstrahl auf der Netzhaut, eine Laserstrahlquelle für die Ausgabe eines Analysestrahls umfassend, der dazu bestimmt ist, auf der Netzhaut des Auges entsprechend einer gegebenen Zielachse fokussiert zu werden, **dadurch gekennzeichnet, dass** es ein System zur Korrektur der Aberrationen des Auges nach einem der Ansprüche 12-18 umfasst, wobei es die Korrektur der Wellenfrontphase des Analysestrahls insbesondere entsprechend der Schätzung der relativen Rotation der Augenachse entgegen einer vorbestimmten Lage ermöglicht.

21. Instrument zur Simulation des Sehvermögens eines Patienten, wobei es ein Befestigungssystem mit einer einen Analysestrahl ausgebenden Lichtquelle sowie ein durch den Analysestrahl beleuchtetes und vom Patienten zu sehendes Testbild umfasst, **dadurch gekennzeichnet, dass** es ein System zur Korrektur der Aberrationen des Auges nach einem der Ansprüche 9-18 umfasst, wobei die Korrektur der Wellenfrontphase des Analysestrahls entsprechend den Bewegungen des Auges ermöglicht wird.

Figure 1

Figure 2A

Figure 2B

EP 1 928 294 B1

Figure 2C

EP 1 928 294 B1

18

**Figure 3**

EP 1 928 294 B1

Figure 4

EP 1 928 294 B1

IMA(1)  T1(1)
T2(1)

**Figure 5A**

IMA(2)  T1(2)
T2(2)

**Figure 5B**

Rar

Rc

A

Rp

Dep.S

Dep.P

**Figure 6**

Figure 7A

Figure 7C

Figure 7B

Figure 7D

**EP 1 928 294 B1**

## RÉFÉRENCES CITÉES DANS LA DESCRIPTION

**Documents brevets cités dans la description**

* FR 0111112